(19) Europäisches Patentamt
European Patent Office
Office européen des brevets

(11) Veröffentlichungsnummer: **0 042 963**
**A1**

(12) **EUROPÄISCHE PATENTANMELDUNG**

(21) Anmeldenummer: **81103788.6**

(22) Anmeldetag: **18.05.81**

(51) Int. Cl.³: **A 01 N 43/08**
C 07 D 307/32

(30) Priorität: **31.05.80 DE 3020763**

(43) Veröffentlichungstag der Anmeldung:
**06.01.82 Patentblatt 82/1**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Behrenz, Wolfgang, Dr.**
**Untergruendemich 14**
**D-5063 Overath(DE)**

(72) Erfinder: **Heine, Hans-Georg, Dr.**
**Am Heckerhof 14**
**D-4150 Krefeld(DE)**

(72) Erfinder: **Lantzsch, Reinhard, Dr.**
**Heymannstrasse 32**
**D-5090 Leverkusen(DE)**

(54) **Tetrahydrofuran-2-on-Derivate enthaltende Schädlingsbekämpfungsmittel, ihre Herstellung und Verwendung.**

(57) Es wurde nun gefunden, daß die neuen Wirkstoffkombinationen aus Tetrahydrofuran-2-on-Derivaten der Formel I

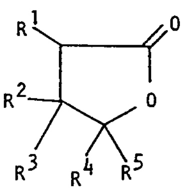

(1)

in welcher

$R^1$ für Wasserstoff, Halogen, Alkyl, Carboxyl oder Alkoxycarbonyl steht,

$R^2$ für Wasserstoff, Alkyl oder Aryl steht,

$R^3$ für Alkyl, für gegebenenfalls durch Halogen und/oder Alkyl substituiertes Cyclopropyl, oder für gegebenenfalls durch Halogen substituiertes Alkenyl steht,

$R^4$ für Wasserstoff oder Alkyl steht und

$R^5$ für Alkyl oder für gegebenenfalls durch Halogen substituiertes Alkenyl steht, und

gegen Arthropoden wirksame Wirkstoffe, vorzugsweise aus der Gruppe der

A) Carbamate,

B) Carbonsäureester, einschließlich der natürlichen sowie synthetischen Pyrethroide,

C) Phosphorester und

D) halogenierten Kohlenwasserstoffe,

als Insektizide verwendet werden können.

EP 0 042 963 A1

BAYER AKTIENGESELLSCHAFT 5090 Leverkusen-Bayerwerk
Zentralbereich
Patente, Marken und Lizenzen S/ABc

# Tetrahydrofuran-2-on-Derivate enthaltende Schädlingsbekämpfungsmittel, ihre Herstellung und Verwendung

Die vorliegende Erfindung betrifft neue Mischungen aus synergistisch wirkenden Tetrahydrofuran-2-on-Derivaten und anderen Wirkstoffen, diese Mischungen enthaltende Schädlingsbekämpfungsmittel, ihre Herstellung und Verwendung zur Bekämpfung von Arthropoden, insbesondere von Insekten, Milben und Spinnentieren.

Es ist bereits bekannt geworden, daß bestimmte Tetrahydrofuran-2-one, z.B. das 5,5-Dimethyl-4-(2',2'-dichlorvinyl)-tetrahydrofuran-2-on oder das 5-(2',2'-Dichlorvinyl)-4,4-dimethyl-tetrahydrofuran-2-on als Zwischenprodukte zur Herstellung von Pyrethroiden, insbesondere von Estern der 2,2-Dimethyl-3-(2',2'-dichlorvinyl)-cyclopropancarbonsäure (Permethrinsäure) verwendet werden (DT-OS 26 30 981).

Aus der US-P 3.488.732 ist bekannt, daß gewisse Dichlorvinyltetrahydrofuran-2-one nematozide Eigenschaften aufweisen.

Ebenfalls bekannt sind insektizide Wirkstoffe bzw. Wirkstoffgruppen der verschiedensten Art, z.B.:



BAD ORIGINAL

A) Carbamate, wie z.B. das 2-iso-Propoxy-phenyl-N-methylcarbamat, 3.4.5-Trimethyl-phenyl-N-methyl-carbamat, 1-Naphthyl-N-methyl-carbamat, 2.3-Dihydro-2,2-dimethyl-7-benzofuranyl-N-methyl-carbamat, 2-(1,3-Dioxolan(2)yl-phenyl)-N-methyl-carbamat und 2,2-Dimethyl-1,3-Benzodioxol(4)yl-N-methyl-carbamat,

B) Carbonsäureester, wie z.B. 2,3,4,5-Tetrahydrophthalimido-methyl-chrysanthemat und (5-Benzyl-3-furyl)-methyl-2,2-dimethyl-3-(2-methylpropenyl)-cyclopropancarboxylat,

C) Phosphorester, wie z.B. O,O-Dimethyl-O-(2,2-dichlorvinyl)-phosphorsäureester und

D) Halogenierte Kohlenwasserstoffe, wie Hexachlorcyclohexan, DDT und Methoxychlor.

Weiterhin sind synergistische Mischungen von Carbamaten, z.B. 2-iso-Propoxy-phenyl-N-methylcarbamat, oder von Phosphorsäureestern, z.B. O,O-Diethyl-O-(2-isopropyl-4-methyl-pyridin-dimethyl[6])-phosphorthioat, oder von natürlichen oder synthetischen Pyrethroiden mit Piperonylethern, wie z.B. α-[2-(2-Butoxy-ethoxy)-ethoxy]-4,5-methylendioxy-2-propyl-toluol, bekannt (vgl. Bull. Org. Health Org. 1966, 35, Seiten 691-708, Schrader, G.: Die Entwicklung neuer insektiziden Phosphorsäureester (1963) S. 158; Perkov, W.: Die Insektizide, 1966, Seiten 516-524). Doch ist die Wirksamkeit dieser synergistischen Wirkstoffkombinationen nicht befriedigend. Eine gewisse praktische Bedeutung hat im wesentlichen bisher nur das α-[2-(2-Butoxy-ethoxy)-ethoxy]-4,5-methylen-

dioxy-2-propyl-toluol erlangt.

Es wurde nun gefunden, daß die neuen Wirkstoffkombinationen aus Tetrahydrofuran-2-on-Derivaten der Formel I

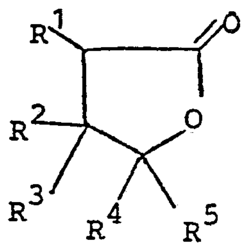

(I)

in welcher

$R^1$ für Wasserstoff, Halogen, Alkyl, Carboxyl oder Alkoxycarbonyl steht,

$R^2$ für Wasserstoff, Alkyl oder Aryl steht,

$R^3$ für Alkyl, für gegebenenfalls durch Halogen und/oder Alkyl substituiertes Cyclopropyl, oder für gegebenenfalls durch Halogen substituiertes Alkenyl steht,

$R^4$ für Wasserstoff oder Alkyl steht und

$R^5$ für Alkyl oder für gegebenenfalls durch Halogen substituiertes Alkenyl steht, und

gegen Arthropoden wirksame Wirkstoffe, vorzugsweise aus der Gruppe der

A) Carbamate,

B) Carbonsäureester, einschließlich der natürlichen sowie synthetischen Pyrethroide,

C) Phosphorester und

D) halogenierten Kohlenwasserstoffe,

wobei diese Wirkstoffe einzeln oder in Mischung vorliegen können,

eine besonders hohe Wirkung gegen Arthropoden, vorzugsweise gegen Insekten, Milben und Spinnentiere, insbesondere gegen Insekten aufweisen.

Die vorliegende Erfindung betrifft somit neue Mischungen aus den synergistisch wirkenden Tetrahydrofuran-2-on-Derivaten der Formel I und anderen gegen Arthropoden wirksamen Wirkstoffen, insbesondere Insektiziden, und diese Mischungen enthaltende Schädlingsbekämpfungsmittel, insbesondere insektizide Mittel, ihre Herstellung sowie die Verwendung der neuen Mischungen und Schädlingsbekämpfungsmittel zur Bekämpfung von Arthropoden, vorzugsweise von Insekten, Milben und Spinnentieren, insbesondere von Insekten.

Die synergistische Wirkung der Verbindungen der allgemeinen Formel I erstreckt sich praktisch auf alle gegen Arthropoden wirksamen Verbindungsklassen. Sie ist bevorzugt bei den folgenden Wirkstoffen von Interesse:

A) Carbamaten der allgemeinen Formel II

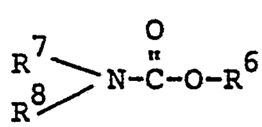

in welcher



BAD ORIGINAL

$R^6$ für Aryl, einen Heterocyclus oder einen Oximrest steht,

$R^7$ für Wasserstoff oder Alkyl mit 1-4 C-Atomen steht und

$R^8$ für Alkyl, Alkylcarbonyl mit 1 bis 6 C-Atomen im Alkylrest, der gegebenenfalls auch durch Hydroxy oder Methylthio substituiert sein kann, oder den Rest -S-Z steht, wobei

Z für gegebenenfalls durch Halogen substituiertes Alkyl mit 1-4 C-Atomen, insbesondere $CCl_3$ und $CF_3$ sowie für gegebenenfalls bevorzugt durch CN, Halogen, insbesondere Chlor, Methyl, Trihalogenmethyl, Trifluormethylmercapto oder $NO_2$ substituiertes Aryl, insbesondere Phenyl, oder für Methoxycarbonyl oder für den Rest $W-SO_2-NR^7-$ steht, wobei W für Alkyl, Halogenalkyl oder Alkylamino, Dialkylamino oder einen gegebenenfalls bevorzugt durch Halogen, Trihalogenmethyl, CN, Methyl oder Nitro substituierten Arylrest steht und $R^7$ die oben angegebene Bedeutung hat.

Unter diesen Verbindungen sind Carbamate bevorzugt, in denen

$R^6$ für Phenyl oder Naphthyl steht, die gegebenenfalls substituiert sind durch Alkyl, Alkenyl, Alkoxy oder Alkylmercapto mit jeweils 1-5 bzw. 2-5 C-Atomen oder durch Dialkylamino, Dialkenylamino mit bis zu 3 C-Atomen je Alkyl- bzw. Alkenylteil oder durch Halogen, insbesondere Chlor, oder durch Dioxolanyl, oder $R^6$ für den Rest $-N{=}CH-N(C_{1-4}\text{-Alkyl})_2$ steht. $R^6$ steht hierbei ganz besonders bevorzugt für durch i-Propoxy oder Dioxolanyl substituiertes Phenyl.



BAD ORIGINAL

Weiterhin sind bevorzugt Carbamate in denen

$R^6$ für 2,3-Dihydrobenzofuranyl, Benzoldioxolyl, Benzothienyl, Pyrimidinyl oder Pyrazolyl steht, die gegebenenfalls durch eine oder mehrere, vorzugsweise 1 oder 2 $C_{1-4}$-Alkylgruppen, insbesondere Methyl oder Dialkylamino mit 1-4 C-Atomen je Alkylteil substituiert sind, wobei der 2,3-Dihydro-2,2-dimethylbenzofuran-7-yl-Rest besonders bevorzugt ist.

Weiterhin sind bevorzugt Carbamate in denen $R^6$ für einen Rest der allgemeinen Formel IIa

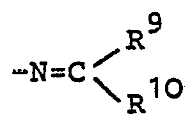

(IIa)

steht, in welcher

$R^9$ und $R^{10}$ gleich oder verschieden sind und für Alkyl, Halogenalkyl (insbesondere t-Butyl und 1,1-Dimethyl-2-fluorethyl), Cycloalkyl, Alkenyl, Alkinyl, Alkoxy, Alkylmercapto, Alkoxycarbonyl, Carbonylamid, Alkylmercaptoalkyl (insbesondere Methylmercaptomethyl) mit jeweils bis zu 5 C-Atomen, CN, Aryl, insbesondere Phenyl, für einen gegebenenfalls substituierten heterocyclischen Rest oder für Alkyl, das durch einen heterocyclischen Rest substituiert ist, stehen oder in welcher $R^9$ und $R^{10}$ gemeinsam einen gegebenenfalls durch $C_{1-4}$-Alkyl substituierten Dioxolanyl oder Dithiolanylrest bilden, wobei besonders bevorzugt $R^9$ für Methyl und $R^{10}$ für Methylthio stehen.

Bei den Carbamaten der Formel II stehen $R^7$ vorzugsweise für Wasserstoff und $R^8$ vorzugsweise für Methyl.

B) Carbonsäureestern der allgemeinen Formel III

$$R^{11}-\overset{O}{\overset{\|}{C}}-O-\overset{R^{12}}{\overset{|}{C}H}-R^{13} \qquad (III)$$

in welcher

$R^{11}$ für Alkyl, Aralkyl, Aryl oder Cycloalkyl steht, die substituiert sein können, und

$R^{12}$ für Wasserstoff, Alkyl, Halogenalkyl, Alkenyl, Alkinyl oder CN steht und

$R^{13}$ für Aryl oder einen Heterocyclus steht, oder in welcher $R^{13}$ gemeinsam mit $R^{12}$ einen gegebenenfalls substituierten Cyclopentenonring bildet.

Bevorzugt sind Carbonsäureester der Formel III in denen $R^{11}$ für Alkyl mit 1-6 C-Atomen, das gegebenenfalls durch gegebenenfalls halogensubstituiertes Phenyl substituiert ist, Cyclopropyl, das gegebenenfalls durch Alkyl, Alkenyl, Halogenalkyl oder Halogenalkenyl mit jeweils bis zu 6 C-Atomen substituiert ist, oder für Phenyl, das gegebenenfalls durch Halogen substituiert ist, steht. Besonders bevorzugt steht hierbei $R^{11}$ für Methyl, für 3-(2,2-Dimethyl- oder 2,2-Dibrom- oder 2,2-Dichlor-vinyl)-2,2-dimethyl-cyclopropyl oder für 2-Methyl-1-(4-chlorphenyl)-propyl(1).

$R^{12}$ steht bevorzugt für Wasserstoff, Alkyl mit 1-6 C-Atomen, Halogenalkyl mit 1-4 C-Atomen und bis zu 3 Halogenatomen, CN oder Ethinyl, insbesondere für Wasserstoff, Trichlormethyl oder CN.



BAD ORIGINAL

$R^{13}$ steht bevorzugt für Phenyl, das gegebenenfalls durch $C_{1-4}$-Alkyl, Halogen, insbesonders Fluor oder Chlor, gegebenenfalls halogen- oder methyl-substituiertes Phenoxy, gegebenenfalls substituiertes Benzyl substituiert ist, ferner für Furanyl, Tetrahydrophthalimido, Benzodioxolyl, die gegebenenfalls durch Halogen, insbesondere Chlor, Alkyl oder Alkenyl mit bis zu 4 C-Atomen oder Benzyl substituiert sind, und ferner für Cyclopentenon, das gegebenenfalls durch $C_{1-4}$-Alkyl, Furfuryl, $C_{2-5}$-Alkenyl substituiert ist. Besonders bevorzugt steht $R^{13}$ für 3,4-Dichlorphenyl, für Pentafluorphenyl, für 5-Benzylfurfuryl, für Tetrahydrophthalimido oder für Phenoxyphenyl, welches im Phenoxyrest oder im Phenylteil durch Halogen, vorzugsweise Fluor, substituiert sein kann.

Weiter sind außer den synthetischen Pyrethroiden auch die natürlich vorkommenden Pyrethroide, wie Pyrethrum, besonders bevorzugt.

C) Phosphor(phosphon)säureestern, einschließlich der Thio- und Thiono-Verbindungen der allgemeinen Formel IV

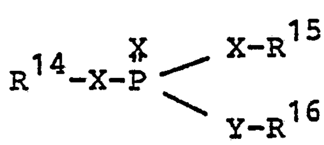

(IV)

in welcher

X unabhängig voneinander für O oder S steht,

Y für O, S, -NH- oder für eine direkte Bindung zwischen dem zentralen P-Atom und dem Rest $R^{16}$ steht,

$R^{14}$ und $R^{15}$ gleich oder verschieden sind und für Alkyl oder Aryl stehen, und

$R^{16}$ für Alkyl, Aryl, Heteroaryl, Aralkyl, Alkenyl, Dioxanyl oder einen Oximrest oder für den gleichen Rest steht, an den es gebunden ist, oder

Y-$R^{16}$ für $NH_2$ steht.

Bevorzugt sind Phosphorsäureester, in denen

$R^{14}$ und $R^{15}$ gleich oder verschieden sind und für $C_{1-4}$, vorzugsweise $C_{1-3}$-Alkyl oder Phenyl stehen,

$R^{16}$ für Alkyl mit 1-4, vorzugsweise 1 oder 2 C-Atomen steht, das gegebenenfalls durch Halogen, vorzugsweise Chlor, OH, CN, gegebenenfalls halogensubstituiertes Phenyl, Carbonylamid, Alkylcarbonylamid, Sulfonylalkyl, Sulfoxyalkyl, Carbonylalkyl, Alkoxy, Alkylmercapto oder Alkoxycarbonyl mit 1 bis 4, vorzugsweise 1 oder 2 Kohlenstoffatomen substituiert ist, für Alkenyl mit bis zu 4 C-Atomen, das gegebenenfalls durch Halogen, vorzugsweise Chlor, gegebenenfalls halogensubstituiertes Phenyl oder Alkoxycarbonyl substituiert ist, oder für den Rest der allgemeinen Formel IV a

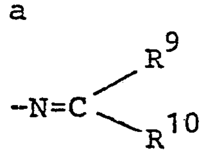

(IV a)

wobei $R^9$ und $R^{10}$ die oben angegebene Bedeutung besitzen, insbesondere jedoch $R^9$ für Cyan und $R^{10}$ für Phenyl stehen, und wobei Y für O steht,



BAD ORIGINAL

$R^{16}$ steht ferner für Dioxanyl, das durch denselben Rest substituiert ist, an den $R^{16}$ gebunden ist, oder $R^{16}$ steht für den gleichen Rest, an den er gebunden ist, oder $R^{16}$ steht für Phenyl, das gegegebenenfalls durch Methyl, Nitro, CN, Halogen, vorzugsweise Chlor, Methylmercapto, Methylsulfonyl und/oder Propoxycarbonyl substituiert ist;

$R^{16}$ steht außerdem bevorzugt für gegebenenfalls durch $C_{1-4}$-Alkyl, Halogen substituierte Reste von Heteroaromaten, wie Pyridin, Chinolin, Chinoxalin, Pyrimidin, Diazinon, Benzo-1,2,4-triazin oder

$Y-R^{16}$ steht für $NH_2$.

Wo nicht jeweils etwas anderes angegeben ist, haben die zu den Formeln I bis IV aufgeführten Definitionen folgende bevorzugte Bedeutung:

Alkyl, Alkylmercapto bzw. Alkylthio, Alkoxy, Alkoxycarbonyl, Halogenalkyl und Alkylsulfonyl enthalten 1 bis 4, insbesondere 1 oder 2 Kohlenstoffatome, Alkenyl, Alkinyl und Halogenalkenylreste enthalten 2 bis 4, insbesondere 2 Kohlenstoffatome.

Aralkyl enthält vorzugsweise 1 bis 3, insbesondere 1 oder 2 Kohlenstoffatome im Alkylteil und Phenyl oder Naphthyl, insbesondere Phenyl im Arylteil, wobei der Benzylrest genannt sei. Alkylamino bzw. Dialkylamino enthält 1 bis 3, vorzugsweise 1 oder 2 Kohlenstoffatome je Alkylgruppe.

Cycloalkyl enthält 3 bis 7, vorzugsweise 3, 5 und 6 Kohlenstoffatome. Aryl bedeutet vorzugsweise Naphthyl oder Phenyl, insbesondere Phenyl.



BAD ORIGINAL

Heterocyclische Reste enthalten 1 oder mehrere Heteroatome, wie Sauerstoff, Schwefel oder Stickstoff, und bestehen aus 5 oder 6 Ringgliedern. Sie können eine oder mehrere Doppelbindungen enthalten. Halogen bedeutet Fluor, Chlor, Brom und Jod, vorzugsweise Fluor, Chlor und Brom, insbesondere Chlor und Fluor.

Substituierte Reste sind ein- oder mehrfach, vorzugsweise 1 bis 3-fach, insbesondere 1- oder 2-fach durch gleiche oder verschiedene Substituenten substituiert.

Überraschenderweise ist insbesondere die insektizide und/oder akarizide Wirkung der erfindungsgemäßen Wirkstoffkombinationen wesentlich höher als die Wirkung der Einzelkomponenten. Sie ist ferner wesentlich höher als die Wirkung der bereits bekannten Wirkstoffkombination aus 2-iso-Propoxy-phenyl-N-methyl-carbamat und Piperonylbutoxid. Außerdem zeigen die als Synergisten erfindungsgemäß verwendbaren Tetrahydrofuran-2-one ausgezeichnete synergistische Wirksamkeit nicht nur bei einer Wirkstoffklasse, sondern bei Wirkstoffen aus den verschiedensten chemischen Stoffgruppen.

Somit stellen die erfindungsgemäßen Tetrahydrofuran-2-on-Derivate enthaltenden synergistischen Mischungen eine wertvolle Bereicherung der Technik dar.

Die für die erfindungsgemäße Kombination zu verwendenden Synergisten sind durch die Formel I allgemein definiert.



BAD ORIGINAL

In der Definition für $R^1$ bis $R^5$ bedeutet Alkyl vorzugsweise geradkettiges oder verzweigtes Alkyl mit 1 bis 4 Kohlenstoffatomen, wie Methyl, Ethyl, n- und i-Propyl und n-, i- und t-Butyl.
Alkoxycarbonyl $R^1$ enthält vorzugsweise 2 bis 4, insbesondere 2 oder 3 Kohlenstoffatome, wie Methoxycarbonyl und Ethoxycarbonyl. Aryl $R^2$ steht vorzugsweise für Naphthyl oder Phenyl, insbesondere für Phenyl.

Cyclopropyl $R^3$ kann ein- oder mehrfach substituiert sein. Vorzugsweise ist es unsubstituiert oder durch 1 bis 3, insbesondere 2 oder 3 gleiche oder verschiedene Substituenten substituiert. Falls es durch Alkyl substituiert ist, enthält jeder Alkylrest vorzugsweise 1 bis 3, insbesondere 1 oder 2 Kohlenstoffatome.

Alkenyl $R^3$ und $R^5$ enthält vorzugsweise 2 bis 5, insbesondere 2 oder 5 Kohlenstoffatome und vorzugsweise 1 Doppelbindung. Es kann durch ein oder mehrere, vorzugsweise 1 bis 3 gleiche oder verschiedene Halogenatome substituiert sein.

Halogen $R^1$ und $R^3$ sowie als Substituent in Cyclopropyl $R^3$ oder in Alkenyl $R^3$ und $R^5$ bedeutet Fluor, Chlor, Brom und Jod, vorzugsweise Fluor, Chlor und Brom, insbesondere Chlor und Brom.

Vorzugsweise stehen in der Formel I jedoch
$R^1$ für Wasserstoff, Carboxyl, für $C_{2-4}$-Alkoxycarbonyl, insbesondere Methoxy-, Ethoxy- und Propoxycarbonyl



BAD ORIGINAL

und Brom,
$R^2$ für Wasserstoff, Methyl oder Ethyl,
$R^3$ für Wasserstoff, für $C_{1-4}$-Alkyl, vorzugsweise Methyl oder Ethyl, für durch Chlor oder Brom substituiertes $C_{2-5}$-Alkenyl oder für durch Chlor, Brom und/oder Methyl substituiertes Cyclopropyl,
$R^4$ für Wasserstoff oder für $C_{1-4}$-Alkyl, vorzugsweise Methyl und Ethyl,
$R^5$ für $C_{1-4}$-Alkyl, vorzugsweise Methyl und Ethyl, oder für durch Chlor oder Brom substituiertes $C_{2-5}$-Alkenyl

Eine besonders gute Wirksamkeit ergeben die Verbindungen der Formel I, in welchen $R^1$ für Wasserstoff steht, $R^2$ für Wasserstoff oder Methyl steht, $R^3$ für 2,2-Dichlorvinyl, für 1,2,2-Trichlorvinyl, für 2,2-Dichlorcyclopropyl oder für 2,2-Dichlor-1-methylcyclopropyl steht, und $R^4$ und $R^5$ für Methyl stehen.

Als Beispiele für erfindungsgemäß verwendbare Tetrahydrofuran-2-on-Derivate der Formel I seien im einzelnen genannt:

4-(2'.2'-Dichlorvinyl)-5.5-dimethyl-tetrahydrofuran-2-on,
4-(2'-Chlorvinyl)-5.5-dimethyl-tetrahydrofuran-2-on,
4-(1'.2'.2'-Trichlorvinyl)-5.5-dimethyl-tetrahydrofuran-2-on,
4-(1'-Chlorvinyl)-5.5-dimethyl-tetrahydrofuran-2-on,
4-(1'.2'.2'-Trichlorvinyl)-4.5.5-trimethyl-tetrahydrofuran-2-on,
4-(2'.2'-Dichlorvinyl)-4.5.5-trimethyl-tetrahydrofuran-2-on.
3-Ethoxycarbonyl-4-(2'.2'-dichlorvinyl)-5.5-dimethyl-tetrahydrofuran-2-on,


BAD ORIGINAL

4-(2'.2'-Dibromvinyl)-5.5-dimethyl-tetrahydrofuran-2-on,
4-(1'.2'.2'-Trichlorvinyl)-5.5-diethyl-tetrahydrofuran-2-on,
4-(1.2'.2'-Trichlorvinyl)-5-ethyl-5-methyl-tetrahydrofuran-2-on,
5-(2'.2'-Dichlorvinyl)-4.4-dimethyl-tetrahydrofuran-2-on,
5-(2'.2'-Dibromvinyl)-4.4-dimethyl-tetrahydrofuran-2-on,
4-(2'.2'-Dichlor-1'-methyl-cyclopropyl)-5.5-dimethyl-tetrahydrofuran-2-on,
4-(2'.2'-Dichlorcyclopropyl)-5.5-dimethyl-tetrahydrofuran-2-on,
4-(2'.2'-Dichlor-1'-methyl-cyclopropyl)-4.5.5-trimethyl-tetrahydrofuran-2-on,
4-(4'-Chlor-2'-methyl-but-3'-en-2'-yl)-5.5-dimethyl-tetrahydrofuran-2-on,
4-(4'.4'-Dichlor-1'-methyl-but-3'-en-2'-yl)-5.5-dimethyl-tetrahydrofuran-2-on,
3-Brom-4-(1'.2'.2'-trichlorvinyl)-5.5-dimethyl-tetrahydrofuran-2-on,
3-n-Propoxycarbonyl-4-(2'.2'-dichlorvinyl)-5.5-dimethyl-tetrahydrofuran-2-on,
4-(2'-Chlor-1'.2'-dibromvinyl)-5.5-dimethyl-tetrahydrofuran-2-on.

Diese Verbindungen sind teilweise neu, können aber nach literaturbekannten Verfahren hergestellt werden (vgl. z.B. DT-OS 2.461.525, 2.509.576, 2.623.777, 2.710.151 und 2.740.849, J.Am.Chem.Soc. 74, 2679 (1952), Pestic. Sci. 1974, 792).



BAD ORIGINAL

Die als Mischkomponenten zu verwendenden Carbamate (Gruppe A) der Formel (II) sind bekannt (vergleiche z.B. US-Patentschriften 3 009 855; 2 903 478 und 3 111 539) und haben sich teilweise bereits als wertvolle Handelsprodukte bewährt. Beispielsweise seien genannt:

2-Methylphenyl-, 2-Ethylphenyl-, 2-n-Propylphenyl-, 2-Methoxyphenyl-, 2-Ethoxyphenyl-, 2-iso-Propoxyphenyl-, 4-Methylphenyl-, 4-Ethylphenyl-, 4-n-Propylphenyl-, 4-Methoxyphenyl-, 4-Ethoxyphenyl-, 4-n-Propoxyphenyl-, 3,4,5-Trimethylphenyl-, 1-Naphthyl-, 2,3-Dihydro-2,2-dimethyl-7-benzofuranyl-, 2-[1,3-Dioxolan(2)yl-phenyl]- bzw. 2,2-Dimethyl-1,3-benzodioxol(4)yl-N-methyl-carbamat und die entsprechenden -N-methyl-N-acetyl-, -N-methyl-N-trifluormethylthio-, -N-methyl-N-dichlormonofluormethylthio- bzw. N-methyl-N-dimethylaminothiocarbamate.

Die weiterhin als Mischungskomponenten verwendbaren Carbonsäureester (Gruppe B) der Formel (III) sind bekannt. Zu ihnen gehören auch die natürlichen und synthetischen Pyrethroide. Als Beispiele seien genannt:

Essigsäure-[1-(3,4-dichlorphenyl)-2,2,2-trichlorethyl]-ester, 2,3,4,5-Tetrahydrophthalimidomethylchrysanthemat und (5-Benzyl-3-furyl)-methyl-2,2-dimethyl-3-(2-methylpropenyl)-cyclopropancarboxylat. (Vergleiche auch R. Wegler "Chemie der Pflanzenschutz- und Schädlingsbekämpfungsmittel" Bd. 1, Seiten 87-118, Heidelberg (1970)).



BAD ORIGINAL

Die ebenfalls als Mischungskomponenten verwendbaren Phosphorverbindungen der Formel (IV) sind bekannt.

Beispielhaft seien genannt:
0,0-Dimethyl- bzw. 0,0-Diethyl-0-(2,2-dichlor- bzw. 2,2-dibromvinyl)-phosphorsäureester. (vergleiche z.B. US-Patentschrift 2 956 073, Deutsche Auslegeschrift 1 167 324, Belgische Patentschrift 633 478).

Einen guten Überblick über erfindungsgemäß verwendbare Carbamate, Carbonsäureester und Phosphorverbindungen liefert auch "Pflanzenschutz und Schädlingsbekämpfung", herausgegeben von K.H. Büchel, Thieme Verlag Stuttgart, 1977).

Wie bereits erwähnt, zeigen die neuen Wirkstoffkombinationen der erfindungsgemäß verwendbaren Tetrahydrofuran-2-on-Derivate der Formel (I) mit Carbamaten, Carbonsäureestern, Phosphorsäureestern und halogenierten Kohlenwasserstoffen eine hervorragende Wirkungssteigerung gegenüber den Einzelwirkstoffen bzw. gegenüber deren Summe.

Die Gewichtsverhältnisse der Wirkstoffgruppen können dabei in relativ großen Bereichen schwanken. Im allgemeinen werden die Tetrahydrofuran-2-on-Derivate mit den übrigen Wirkstoffen im Verhältnis 0,1:10 bis 10:0,1 eingesetzt. Besonders geeignet haben sich jedoch Mischungsverhältnisse von 0,5:1,0 bis 3,0:1,0 erwiesen. Die erfindungsgemäßen Wirkstoffkombinationen bewirken nicht nur eine schnelle knock-down-Wirkung, sondern bewirken auch die nachhaltige Abtötung aller



BAD ORIGINAL

oder einzelner Entwicklungsstadien tierischer Schädlinge, insbesondere Insekten. Zu den Schädlingen gehören diejenigen, die in der Landwirtschaft, in Forsten, im Vorrats- und Materialschutz, sowie auf dem Hygienesektor vorkommen. Dazu gehören:

Aus der Ordnung der Isopoda z.B. Oniscus asellus, Porcellio scaber.
Aus der Ordnung der Thysanura z.B. Lepisma saccharina.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Heteroptera z.B. Cimex lectularius, Rhodnius prolixus, Triatoma spp.
Aus der Ordnung der Homoptera z.B. Myzus spp., und Psylla spp.
Aus der Ordnung der Lepidoptera z.B. Ephestia kuehniella und Galleria mellonella.
Aus der Ordnung der Coleoptera z.B. Anobium punctatum, Rhizopertha dominica, Bruchidius obtectus, Acanthoscelides obtectus, Hylotrupes bajulus, Oryzaephilus surinamensis, Sitophilus spp., Dermestes spp., Trogoderma spp., Anthrenus spp., Attagenus spp., Lyctus spp.,



BAD ORIGINAL

Ptinus spp., Niptus hololeucus, Gibbium psylloides, Tribolium spp. und Tenebrio molitor.
Aus der Ordnung der Hymenoptera z.B. Lasius spp., Monomorium pharaonis, Vespa spp.
Aus der Ordnung der Diptera z.B. Aädes spp., Anopheles spp., Culex spp., Drosophila melanogaster, Musca spp., Fannia spp., Calliphora erythrocephala, Lucilia spp., Chrysomyia spp., Gastrophilus spp., Hyppobosca spp., Stomoxys spp., Oestrus spp., Hypoderma spp. und Tabanus spp.
Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Boophilus spp., Rhipicephalus spp., Amblyomma spp., Hyalomma spp., Ixodes spp., Psoroptes spp., Chorioptes spp., Sarcoptes spp.

Die Wirkstoffkombinationen können in die üblichen Formulierungen übergeführt werden, wie Lösungen, Emulsionen, Spritzpulver, Suspensionen, Pulver, Stäubemittel, Schäume, Pasten, lösliche Pulver, Aerosole, Suspensions-Emulsionskonzentrate, Wirkstoff-imprägnierte Natur- und synthetische Stoffe, Feinstverkapselungen in polymeren Stoffen, ferner in Formulierungen mit Brennsätzen, wie Räucherpatronen, -dosen, -spiralen u.ä., sowie ULV-Kalt- und Warmnebel-Formulierungen.



BAD ORIGINAL

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkchole, wie Butanol oder Glycol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe



BAD ORIGINAL

für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe, und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90 %.

Die Anwendung der erfindungsgemäßen Wirkstoffkombinationen erfolgt in Form ihrer handelsüblichen Formu-



BAD ORIGINAL

lierungen und/oder den aus diesen Formulierungen bereiteten Anwendungsformen.

Der gesamte Wirkstoffgehalt der aus den handelsüblichen Formulierungen bereiteten Anwendungsformen kann in weiten Bereichen variieren. Die Wirkstoffkonzentration der Anwendungsformen kann von 0,0001 bis zu 100 Gew.-% Wirkstoffkombination, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Die folgenden Beispiele zeigen die Wirkstoffe und die erfindungsgemäß verwendbaren Tetrahydrofuran-2-on-Derivate, bei deren Kombination beispielshaft eine synergistische Wirkung nachgewiesen wurde:

I. Beispiele für die erfindungsgemäß verwendbaren Wirkstoffe

A) 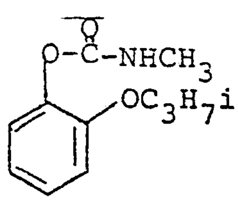 (Propoxur)



BAD ORIGINAL

B) 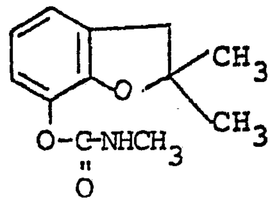 (Carbofuran)

C) 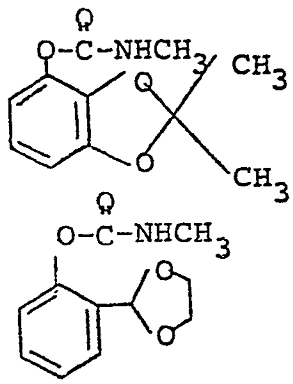 (Bendiocarb)

D) (Dioxacarb)

E) 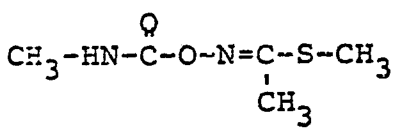 (Methomyl)

F) Pyrethrine natürlischer Herkunft als 25 %iger Extrakt

G) 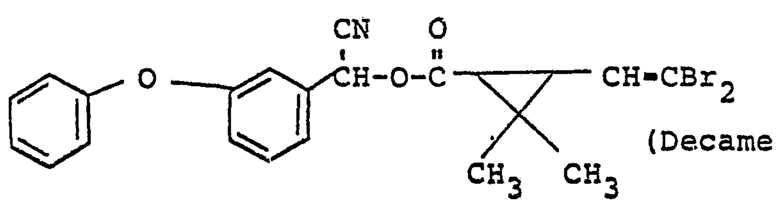 (Decamethrin)

H) 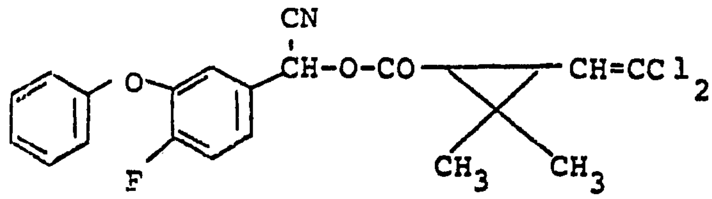

I) 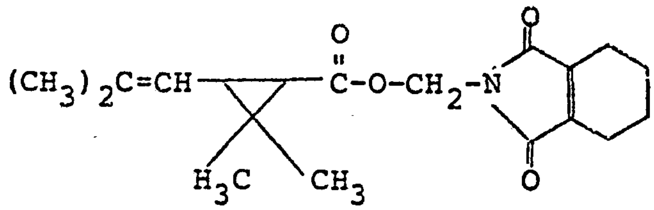 (Tetramethrin)

K) 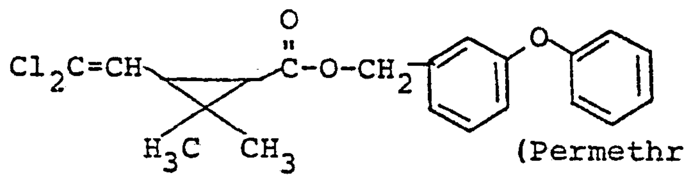

(Permethrin)

L) 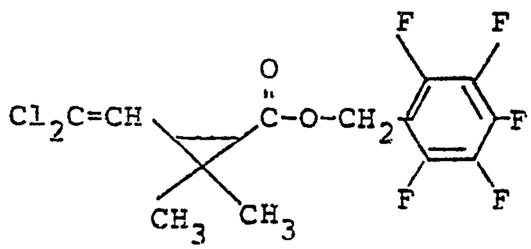

M) 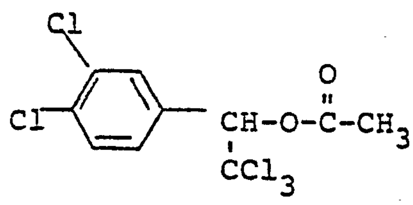 (Penfenate)

N) 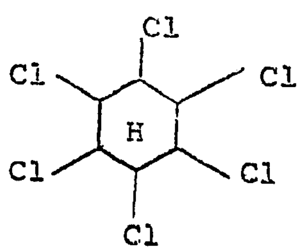 (γ-Hexachlorcyclohexan)

O) $CCl_2{=}CH{-}O{-}\overset{O}{\overset{\|}{P}}{-}(OCH_3)_2$

(DDVP)

P) $CH_3HN{-}\overset{O}{\overset{\|}{C}}{-}CH_2{-}S{-}\overset{O}{\overset{\|}{P}}(OCH_3)_2$

(Omethoat)

II. Beispiele von erfindungsgemäß verwendbaren Synergisten

1)

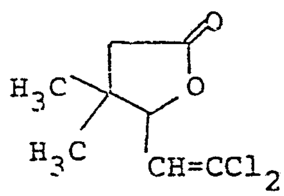

2)

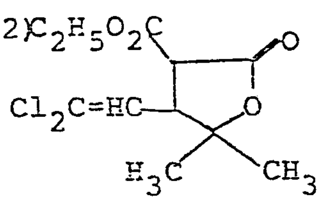

3)

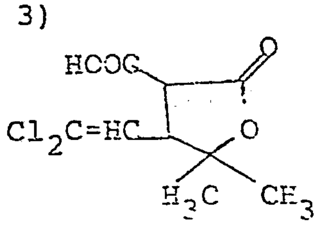

4)

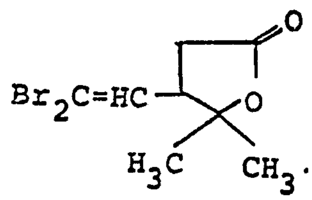

5)

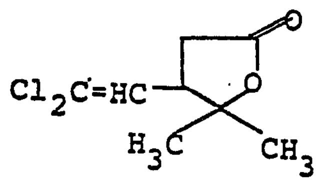

6)

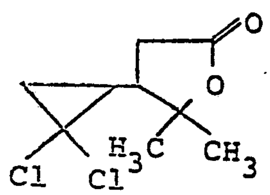

7)

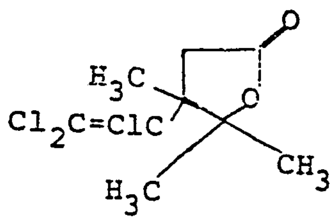

8)

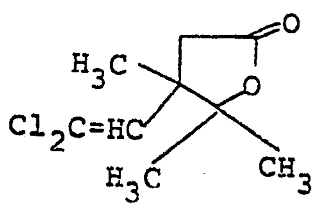

9)

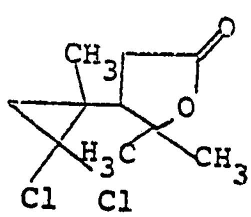

10) 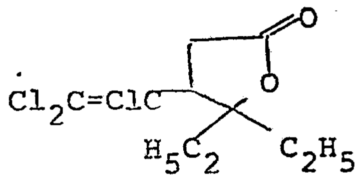

11) 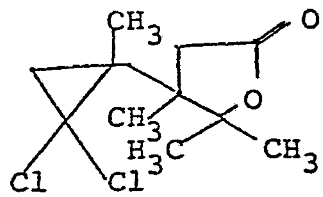

12) 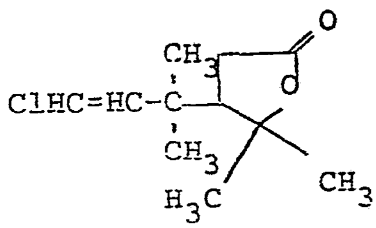

13) 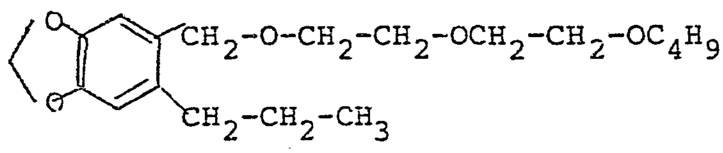

Piperonylbutoxid (bekannt)

In den folgenden Tabellen werden die bekannten Wirkstoffe mit den voranstehend angegebenen Großbuchstaben gekennzeichnet, während für die erfindungsgemäß verwendbaren Synergisten die vorstehend angegebenen Ziffern verwendet werden.



BAD ORIGINAL

Beispiel

$LT_{100}$-Test

Testtiere: Gegen Phosphorsäureester resistente weibliche Musca domestica (Stamm Weymanns)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipettiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100 %ige knock-down-Wirkung erforderlich ist. Wird die $LT_{100}$ nach 6 Stunden nicht erreicht, wird der Prozent der knock-down gegangenen Testtiere festgestellt.

Konzentrationen der Wirkstoffe, Synergisten und Gemische und ihre Wirkungen gehen aus der nachfolgenden Tabelle hervor.



BAD ORIGINAL

Tabelle

LT-100 Test mit gegen Phosphorsäureester resistenten weiblichen Musca domestica (Stamm Weymanns)

| Wirkstoffe/Synergisten | | Konzentrationen in % Wirkstoff/Synergist | | LT 100 in Minuten oder bei 360' in % |
|---|---|---|---|---|
| A | | 1.0 | | 360'= 0% |
| B | | 1.0 | | 360'= 0% |
| C | | 1.0 | | 360'= 10% |
| D | | 1.0 | | 360'= 0% |
| E | | 0.2 | | 360'= 95% |
| F | | 0.2 | | 120' |
| G | | 0.0016 | | 150' |
| H | | 0.008 | | 105' |
| I | | 0.2 | | 105' |
| K | | 0.04 | | 150' |
| L | | 0.0016 | | 60' |
| M | | 1.0 | | 360'= 70% |
| N | | 0.2 | | 360'= 95% |
| O | | 0.008 | | 180' |
| P | | 0.2 | | 360'= 40% |
| | 1 | | 1.0 | 360'= 60% |
| | 2 | | 1.0 | 360'= 30% |
| | 3 | | 1.0 | 360'= 0% |
| | 4 | | 1.0 | 360'= 15% |
| | 5 | | 1.0 | 360'= 30 % |
| | 6 | | 1.0 | 360'= 70% |
| | 7 | | 1.0 | 360'= 35% |
| | 8 | | 1.0 | 360'= 90% |



BAD ORIGINAL

| Wirstoffe/Synergisten | Konzentrationen in % Wirkstoff/Synergist | LT 100 in Minuten oder bei 360' in % |
|---|---|---|
| 9 | 1,0 | 360' = 40 % |
| 10 | 0,2 | 360' = 40 % |
| 11 | 1,0 | 360' = 70 % |
| 12 | 1,0 | 360' = 65 % |
| 13 | 1,0 | 360' = 0 % |

Tabelle

LT 100-Test mit phosphorsäureesterresistenten weiblichen Musca domestica (Stamm Weymanns)

| Wirkstoff + Synergist | Konzentration in % Wirkstoff + Synergist | LT-100 in Minuten oder bei 360' in % |
|---|---|---|
| A + 13 | 1,0 + 1,0 | 360'= 80 % |
| A + 1 | 0,2 + 0,2 | 150' |
| A + 2 | 0,2 + 0,2 | 360' |
| A + 4 | 0,04 + 0,04 | 240' |
| A + 6 | 0,04 + 0,04 | 150' |
| A + 7 | 0,008 + 0,008 | 240' |
| A + 8 | 0,04 + 0,04 | 90' |
| A + 9 | 0,04 + 0,04 | 120' |
| A + 11 | 0,04 + 0,04 | 210' |
| A + 12 | 0,04 + 0,04 | 210' |
| B + 13 | 0,2 + 0,2 | 360' = 50 % |
| B + 1 | 0,04 + 0,04 | 210' |
| B + 2 | 0,04 + 0,04 | 180' |
| B + 4 | 0,04 + 0,04 | 180' |
| B + 5 | 0,008 + 0.008 | 210' |
| B + 6 | 0,04 + 0,04 | 105' |
| B + 7 | 0,008 + 0,008 | 120' |
| B + 8 | 0,008 + 0,008 | 180' |
| B + 9 | 0,008 + 0,008 | 240' |
| B + 10 | 0,2 + 0,2 | 120' |
| B + 11 | 0,04 + 0,04 | 150' |
| B + 12 | 0,2 + 0,2 | 210' |



BAD ORIGINAL

Tabelle Forts.

LT 100-Test mit phosphorsäureresistenten weiblichen Musca domestica (Stamm Weymanns)

| Wirkstoff + Synergist | Konzentration in % Wirkstoff + Synergist | LT-100 in Minuten oder bei 360' in % |
|---|---|---|
| C + 13 | 0,2 + 0,2 | 360' = 85 % |
| C + 1 | 0,04 + 0,04 | 150' |
| C + 2 | 0,04 + 0,04 | 150' |
| C + 4 | 0,008 + 0,008 | 180' |
| C + 5 | 0,008 + 0,008 | 210' |
| C + 6 | 0,008 + 0,008 | 360' |
| C + 7 | 0,008 + 0,008 | 150' |
| C + 8 | 0,008 + 0,008 | 150' |
| C + 9 | 0,008 + 0,008 | 360' |
| C + 10 | 0,2 + 0,2 | 105' |
| C + 11 | 0,04 + 0,04 | 105' |
| C + 12 | 0,04 + 0,04 | 90' |
| D + 13 | 1,0 + 1,0 | 360' = 95 % |
| D + 1 | 0,2 + 0,2 | 210' |
| D + 2 | 0,2 + 0,2 | 360' |
| D + 4 | 0,2 + 0,2 | 210' |
| D + 5 | 0,2 + 0,2 | 150' |
| D + 6 | 0,2 + 0,2 | 180' |
| D + 7 | 0,2 + 0,2 | 360' |
| D + 8 | 0,04 + 0,04 | 210' |
| D + 9 | 0,2 + 0,2 | 360' |
| D + 10 | 1,0 + 1,0 | 105' |
| D + 11 | 0,2 + 0,2 | 210' |
| D + 12 | 0,2 + 0,2 | 210' |



BAD ORIGINAL

Tabelle Forts.

LT 100-Test mit phosphorsäureresistenten weiblichen Musca domestica (Stamm Weymanns)

| Wirkstoff + Synergist | Konzentration in % Wirkstoff + Synergist | LT 100 in Minuten oder bei 360' in % |
|---|---|---|
| E + 13 | 0,2 + 0,2 | 150' |
| E + 1 | 0,2 + 0,2 | 90' |
| E + 2 | 0,2 + 0,2 | 90' |
| E + 4 | 0,2 + 0,2 | 75' |
| E + 5 | 0,04 + 0,04 | 105' |
| E + 6 | 0,2 + 0,2 | 90' |
| E + 7 | 0,04 + 0,04 | 105' |
| E + 8 | 0,04 + 0,04 | 105' |
| E + 9 | 0,04 + 0,04 | 120' |
| E + 10 | 0,2 + 0,2 | 90' |
| E + 11 | 0,2 + 0,2 | 90' |
| E + 12 | 0,2 + 0,2 | 105' |
| F + 1 | 0,04 + 0,04 | 75' |
| F + 2 | 0,2 + 0,2 | 60' |
| F + 4 | 0,2 + 0,2 | 75' |
| F + 5 | 0,04 + 0,04 | 90' |
| F + 6 | 0,04 + 0,04 | 75' |
| F + 7 | 0,04 + 0,04 | 90' |
| F + 8 | 0,04 + 0,04 | 90' |
| F + 9 | 0,04 + 0,04 | 75' |
| F + 10 | 0,2 + 0,2 | 45' |
| F + 11 | 0,04 + 0,04 | 90' |
| F + 12 | 0,04 + 0,04 | 105' |



BAD ORIGINAL

Tabelle Forts.

LT 100-Test mit phosphorsäureresistenten weiblichen Musca domestica (Stamm Weymanns)

| Wirkstoff + Synergist | Konzentration in % Wirkstoff + Synergist | LT 100 in Minuten oder bei 360' in % |
|---|---|---|
| G + 1 | 0,0016 + 0,0016 | 105' |
| G + 2 | 0,0016 + 0,0016 | 105' |
| G + 3 | 0,0016 + 0,0016 | 90' |
| G + 4 | 0,0016 + 0,0016 | 90' |
| G + 5 | 0,0016 + 0,0016 | 105' |
| G + 6 | 0,0016 + 0,0016 | 105' |
| G + 7 | 0,0016 + 0,0016 | 120' |
| G + 8 | 0,0016 + 0,0016 | 105' |
| G + 9 | 0,0016 + 0,0016 | 90' |
| G + 10 | 0,0016 + 0,0016 | 90' |
| G + 11 | 0,0016 + 0,0016 | 90' |
| G + 12 | 0,0016 + 0,0016 | 105' |
| | | |
| H + 2 | 0,008 + 0,008 | 90' |
| H + 4 | 0,008 + 0,008 | 90' |
| H + 5 | 0,008 + 0,008 | 90' |
| H + 6 | 0,008 + 0,008 | 75' |
| H + 8 | 0,008 + 0,008 | 90' |
| H + 9 | 0,008 + 0,008 | 75' |
| H + 10 | 0,008 + 0,008 | 90' |
| H + 11 | 0,008 + 0,008 | 75' |
| H + 12 | 0,008 + 0,008 | 90' |

Tabelle Forts.

LT 100-Test mit phosphorsäureresistenten weiblichen Musca domestica (Stamm Weymanns)

| Wirkstoff + Synergist | Konzentration in % Wirkstoff | + Synergist | LT 100 in Minuten, oder bei 360' in % |
|---|---|---|---|
| J + 1 | 0,2 | + 0,2 | 45' |
| J + 2 | 0,2 | + 0,2 | 75' |
| J + 4 | 0,2 | + 0,2 | 75' |
| J + 5 | 0,2 | + 0,2 | 60' |
| J + 6 | 0,2 | + 0,2 | 60' |
| J + 8 | 0,2 | + 0,2 | 60' |
| J + 9 | 0,2 | + 0,2 | 90' |
| J + 10 | 0,2 | + 0,2 | 45' |
| J + 11 | 0,2 | + 0,2 | 90' |
| J + 12 | 0,04 | + 0,04 | 75' |
| K + 1 | 0,04 | + 0,04 | 75' |
| K + 2 | 0,04 | + 0,04 | 75' |
| K + 4 | 0,04 | + 0,04 | 90' |
| K + 5 | 0,04 | + 0,04 | 75' |
| K + 6 | 0,04 | + 0,04 | 75' |
| K + 7 | 0,04 | + 0,04 | 90' |
| K + 8 | 0,04 | + 0,04 | 90' |
| K + 9 | 0,04 | + 0,04 | 90' |
| K + 10 | 0,04 | + 0,04 | 90' |
| K + 11 | 0,04 | + 0,04 | 90' |
| K + 12 | 0,04 | + 0,04 | 75' |
| L + 13 | 0,0016 | + 0,0016 | 75' |
| L + 8 | 0,0016 | + 0,0016 | 45' |



BAD ORIGINAL

Tabelle Forts.

LT 100-Test mit phosphorsäureresistenten weiblichen Musca domestica (Stamm Weymanns)

| Wirkstoff + Synergist | Konzentration in % Wirkstoff + Synergist | | LT 100 in Minuten, oder bei 360' in % |
|---|---|---|---|
| M + 13 | 1,0 | + 1,0 | 360' = 85 % |
| M + 1 | 0,2 | + 0,2 | 360' |
| M + 6 | 0,04 | + 0,04 | 360' = 90 % |
| M + 7 | 0,04 | + 0,04 | 360' = 95 % |
| M + 8 | 0,04 | + 0,04 | 360' = 95 % |
| M + 9 | 0,04 | + 0,04 | 360' = 95 % |
| M + 10 | 0,2 | + 0,2 | 360' = 85 % |
| M + 11 | 0,04 | + 0,04 | 360' |
| M + 12 | 0,2 | + 0,2 | 360' |
| N + 13 | 0,2 | + 0,2 | 240' |
| N + 1 | 0,2 | + 0,2 | 150' |
| N + 2 | 0,2 | + 0,2 | 120' |
| N + 3 | 0,2 | + 0,2 | 150' |
| N + 4 | 0,2 | + 0,2 | 180' |
| N + 5 | 0,04 | + 0,04 | 120' |
| N + 6 | 0,04 | + 0,04 | 150' |
| N + 7 | 0,008 | + 0,008 | 180' |
| N + 8 | 0,04 | + 0,04 | 180' |
| N + 9 | 0,04 | + 0,04 | 150' |
| N + 10 | 0,2 | + 0,2 | 180' |
| N + 11 | 0,2 | + 0,2 | 210' |
| N + 12 | 0,04 | + 0,04 | 180' |



BAD ORIGINAL

Tabelle Forts.

LT 100-Test mit phosphorsäureresistenten weiblichen Musca domestica (Stamm Weymanns)

| Wirkstoff + Synergist | Konzentration in % Wirkstoff + Synergist | LT 100 in Minuten; oder bei 360' in % |
|---|---|---|
| O + 13 | 0,008 + 0,008 | 120' |
| O + 1 | 0,008 + 0,008 | 90' |
| O + 2 | 0,008 + 0,008 | 75' |
| O + 4 | 0,008 + 0,008 | 60' |
| O + 5 | 0,008 + 0,008 | 60' |
| O + 6 | 0,008 + 0,008 | 60' |
| O + 7 | 0,008 + 0,008 | 60' |
| O + 8 | 0,008 + 0,008 | 60' |
| O + 9 | 0,008 + 0,008 | 75' |
| O + 11 | 0,008 + 0,008 | 75' |
| O + 12 | 0,008 + 0,008 | 90' |
| P + 13 | 0,2 + 0,2 | 240' |
| P + 1 | 0,2 + 0,2 | 90' |
| P + 2 | 0,2 + 0,2 | 150' |
| P + 3 | 0,2 + 0,2 | 180' |
| P + 4 | 0,2 + 0,2 | 210' |
| P + 5 | 0,2 + 0,2 | 105' |
| P + 6 | 0,2 + 0,2 | 180' |
| P + 7 | 0,2 + 0,2 | 210' |
| P + 8 | 0,04 + 0,04 | 180' |
| P + 9 | 0,2 + 0,2 | 120' |
| P + 10 | 0,2 + 0,2 | 120' |
| P + 11 | 0,04 + 0,04 | 210' |
| P + 12 | 0,2 + 0,2 | 120' |



BAD ORIGINAL

Die Herstellung der erfindungsgemäß verwendbaren Synergisten ist bekannt, Die Herstellung einiger dieser Verbindungen wird der Einfachheit halber in den folgenden Herstellungsbeispielen angegeben:

Herstellungsbeispiele:

Beispiel 1 (Verbindung 12)

Die Lösung von 34.1 g (0.17 mol) 3-(4'-Chlor-2'-methylbut-3'-en-2'-yl)-2.2-dimethyl-cyclobutanon in 380 ml Eisessig wird bei 15-20°C innerhalb von 30 min. mit 100 ml 30 proz. Wasserstoffperoxid versetzt. Nach 20-stündigem Rühren gießt man auf Eis, extrahiert mit Methylenchlorid, trocknet über wasserfreiem Natriumsulfat und dampft die Methylenchloridphase ein. Fraktionierende Destillation des Rückstands (39,9 g) liefert 30.7 g 4-(4'-Chlor-2'-methyl-but-3'-en-2'-yl)-5.5-dimethyl-tetrahydrofuran-2-on vom Siedepunkt 0,08-0.1 (mm Hg) 110-115°C.

Beispiel 2

30.65 g (0.1 mol) 4-Brom-2.2-dimethyl-3-(1'.2'.2'-trichlorvinyl)-cyclobutanon werden in 225 ml Eisessig mit 57.5 ml 30 proz. Wasserstoffperoxid bei 15-20°C innerhalb von 15 min. versetzt. Nach 20stündigem Rühren bei 20°C gibt man den Ansatz auf 1000 ml Eiswasser und extrahiert mit Methylenchlorid.
Übliches Aufarbeiten liefert 29.7 g Rohprodukt, das aus 70 ml Cyclohexan kristallisiert wird. 20.8 g 3-Brom-5.5-dimethyl-4-(1'.2'.2'-trichlorvinyl)-tetrahydrofuran-2-on



BAD ORIGINAL

vom Schmelzpunkt 110-11°C werden erhalten.

Beispiel 3

15.8 g (0.1 mol) 3-(1'-Chlorvinyl)-2.2-dimethyl-cyclobutanon werden in 200 ml Eisessig bei 15-20°C mit 25 ml 30 proz. Wasserstoffperoxid versetzt. Nach 15-stündigem Rühren bei 20°C wird, wie in Beispiel 2 beschrieben, aufgearbeitet. 10.0 g 4-(1'-Chlorvinyl)-5.5-dimethyltetrahydrofuran-2-on vom Siedepunkt $_{0.06-0.1\ (mm\ Hg)}$ 64-68°C $n_D^{20}$ 1.4821 werden erhalten.

Beispiel 4 (Verbindung 11)

35.3 g (0.15 mol) 3-(2'.2'-Dichlor-1'-methylcyclopropyl)-2.2.3-trimethyl-cyclobutanon werden in 310 ml Eisessig mit 80 ml 30 proz. Wasserstoffperoxid bei 15-20°C innerhalb von 40 min. tropfenweise versetzt. Nach 20-stündigem Rühren bei 20°C werden 1500 ml Wasser zugesetzt. Extrahieren mit Methylenchlorid, Trocknen der org. Phase über wasserfreiem Natriumsulfat und Einengen in Vakuum liefern 39.1 g farblose Kristalle, die zweimal aus je 50 ml Cyclohexan kristallisiert werden. Schmelzpunkt 100-102°C.

Beispiel 5 (Verbindung 9)

4-(2'.2'-Dichlor-1'-methyl-cyclopropyl)-5.5-dimethyltetrahydrofuran-2-on wird analog Beispiel 4 erhalten. Schmelzpunkt 82-86°C.



BAD ORIGINAL

Beispiel 6 (Verbindung 6)

4-(2'.2'-Dichlorcyclopropyl)-5.5-dimethyl-tetrahydrofuran-2-on wird analog Beispiel 4 erhalten. Schmelzpunkt 84-85°C.

Beispiel 7

4-(2'-Chlor-1'.2'-dibromvinyl)-5.5-dimethyl-tetrahydrofuran-2-on

Zu dem Gemisch von 960.0 g (4.0 mol) 3.5.5.5-Tetrachlor-2-methyl-pentan-2-ol und 40.0 g Tetrabutylammoniumbromid tropft man innerhalb von 2 h unter Rühren 1077.0 g (12.12 mol) 45 proz. Natronlauge. Die leicht exotherme Reaktion führt zu einer Temperaturerhöhung auf 60°C. Anschließend erhitzt man die Reaktionslösung 5 h auf 100°C. Nach dem Abkühlen setzt man 2000 ml Wasser zu und neutralisiert durch Zugabe von 10 proz. Salzsäure. Die organische Phase wird abgetrennt und über eine 20 cm lange Vigreux-Kolonne fraktionierend destilliert. 355.0 g 2.2-Dimethyl-3-chlorethinyloxiran vom Siedepunkt 45-50°C/15 mmHg werden erhalten.

131.0 g 2.2-Dimethyl-3-chlorethinyl-oxiran in 500 ml Methylenchlorid werden unter Kühlung mit 51 ml Brom in 250 ml Methylenchlorid bei 20°C innerhalb von 8 h versetzt. Nach Rühren über Nacht wird fraktionierend destilliert. Man erhält 187.0 g 3-(2-Chlor-1.2-dibromvinyl)2.2-dimethyl-oxiran vom Siedepunkt 48-51°C/0,2 mmHg.



BAD ORIGINAL

Zu der Lösung von 54.0 g (1.0 mol) Natriummethylat in 400 ml Ethanol werden innerhalb von 30 Min. 160.0 g (1.0 mol) Malonsäurediethylester getropft. Anschließend gibt man bei 30-35°C innerhalb von 1 h insgesamt 296.0 g (1.2 mol) 3-(2-Chlor-1.2-dibromvinyl)-2.2-dimethyl-oxiran zu. Darauf rührt man das Reaktionsgemisch 5 h bei 40°C. Nach Kühlen auf 20°C setzt man 500,0 g Eiswasser und 500,0 g 25 proz. Schwefelsäure zu und destilliert Lösungsmittel und einen Teil des zugesetzten Wassers soweit ab, bis die Sumpftemperatur auf 117°C angestiegen ist. Danach wird das Gemisch 5 h bei 117°C gerührt. Nach Kühlen auf 20°C werden 1000 ml Wasser zugesetzt. Man extrahiert mit Methylenchlorid, trocknet den Methylenchloridextrakt über wasserfreiem Natriumsulfat und destilliert fraktionierend. 210.0 g 4-(2'-Chlor-1'.2'-dimbromvinyl)-5.5-dimethyl-tetrahydrofuran-2-on vom Siedepunkt 80-82°C / 0.05 mmHg, Schmelpunkt 112-114°C (Ethanol) werden erhalten.

Wie bereits oben dargelegt, können auch die übrigen erfindungsgemäß verwendbaren Verbindungen der Formel I nach bekannten Methoden erhalten werden.



BAD ORIGINAL

Patentansprüche

1. Gegen Arthropoden wirksame Mischungen, welche wenigstens einen gegen Arthropoden wirksamen Wirkstoff, vorzugsweise aus der Gruppe der Carbamate, der Ester, der Phosphorverbindungen oder der halogenierten Kohlenwasserstoffe, und wenigstens einen Synergisten enthalten, dadurch gekennzeichnet, daß der Synergist ein Tetrahydrofuran-2-on Derivat der Formel I

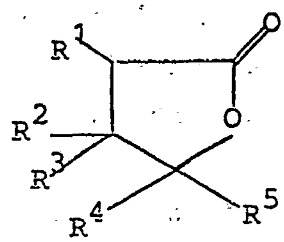

(I)

ist,

in welcher,

$R^1$ für Wasserstoff, Halogen, Alkyl, Carboxyl oder Alkoxycarbonyl steht,

$R^2$ für Wasserstoff, Alkyl oder Aryl steht,

$R^3$ für Alkyl, für gegebenenfalls durch Halogen und/oder Alkyl substituiertes Cyclopropyl oder für gegebenenfalls durch Halogen substituiertes Alkenyl steht,

$R^4$ für Wasserstoff oder Alkyl steht und

$R^5$ für Alkyl oder für gegebenenfalls durch Halogen substituiertes Alkenyl steht.

2. Mischungen gemäß Anspruch 1, in welchen bei dem Tetrahydrofuran-2-on-Derivat der Formel I



BAD ORIGINAL

$R^1$ für Wasserstoff, Carboxyl, für Alkoxycarbonyl mit 2-4 Kohlenstoffatomen oder für Brom steht,

$R^2$ für Wasserstoff, Methyl oder Ethyl steht,

$R^3$ für Alkyl mit 1 bis 4 Kohlenstoffatomen, für durch Chlor oder Brom substituiertes Alkenyl mit 2 bis 5 Kohlenstoffatomen oder für durch Chlor, Brom und/oder Methyl substituiertes Cyclopropyl steht,

$R^4$ für Wasserstoff oder für Alkyl mit 1 bis 4 Kohlenstoffatomen steht und

$R^5$ für Alkyl mit 1 bis 4 Kohlenstoffatomen oder für durch Chlor oder Brom substituiertes Alkenyl mit 2 bis 5 Kohlenstoffatomen steht.

3. Mischungen gemäß Anspruch 1, in welchen bei dem Tetrahydrofuran-2-on-Derivat der Formel I

$R^1$ für Wasserstoff; Ethoxycarbonyl und Carboxyl steht,

$R^2$ für Wasserstoff oder Methyl steht,

$R^3$ für 2,2-Dichlorvinyl, für 2,2-Dibromvinyl, für 1,2,2,-Trichlorvinyl, für 2,2-Dichlorcyclopropyl, für 4-Chlor-2-methyl-but-3-en-2-yl oder für 2,2-Dichlor-1-methyl-cyclopropyl steht und

$R^4$ für Wasserstoff oder Methyl steht und

$R^5$ für Methyl, für 2,2-Dichlorvinyl oder für 1,2,2-Trichlorvinyl steht.

4. Mischungen gemäß Anspruch 1, in welchen bei dem Tetrahydrofuran-2-on-Derivat der Formel I



BAD ORIGINAL

$R^1$ für Wasserstoff steht,
$R^2$ für Wasserstoff oder Methyl steht,
$R^3$ für 2,2-Dichlorvinyl, für 1,2,2-Trichlorvinyl, für 2,2-Dichlorcyclopropyl oder für 2,2-Dichlor-1-methylcyclopropyl steht und
$R^4$ und $R^5$ für Methyl stehen.

5. Mischungen gemäß den Ansprüchen 1 bis 4, in welchen das Gewichtsverhältnis von Wirkstoff zu Synergisten zwischen 1:10 und 10:1 liegt.

6. Gegen Arthropoden wirksame Mittel, gekennzeichnet durch einen Gehalt an einer Mischung gemäß den Ansprüchen 1 bis 5.

7. Verfahren zur Herstellung der Mittel gemäß Anspruch 6, dadurch gekennzeichnet, daß man Mischungen gemäß den Ansprüchen 1 bis 5 mit inerten Träger- und Verdünnungsstoffen, Füllstoffen, Treibgasen, oberflächenaktiven Stoffen und/oder Formulierhilfsmitteln mischt.

8. Verwendung der Mischungen gemäß den Ansprüchen 1 bis 5 zur Bekämpfung von Arthropoden, insbesondere Insekten, Milben und Spinnentieren.

9. Verwendung der Mittel gemäß Anspruch 6 zur Bekämpfung von Arthropoden, insbesondere Insekten, Milben und Spinnentieren.

10. Verwendung der Mischungen gemäß den Ansprüchen 1 bis 5 und der Mittel gemäß Anspruch 6 zur Bekämpfung von Insekten.



BAD ORIGINAL

0042963

Europäisches Patentamt

# EUROPÄISCHER RECHERCHENBERICHT

Nummer der Anmeldung
EP 81 10 3788

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | CENTRAL PATENTS INDEX, Basic Abstracts Journal, Section C-Ag-Doc week 34, abstract 60328y<br>& JP - A - 77 83457 (KURARAY K.K.)<br>* Zusammenfassung *<br>-- | 1-10 | A 01 N 43/08<br>C 07 D 307/32 |
| | CENTRAL PATENTS INDEX, Basis Abstracts Journal, Section C-Ag-Doc week 34, abstract 60474y<br>& JP - A - 77 83734 (KURARAY K.K.)<br>* Zusammenfassung *<br>-- | 1-10 | RECHERCHIERTE SACHGEBIETE (Int. Cl.³) |
| | EP - A - 0 001 089 (BAYER A.G.)<br>* Seite 9 *<br>-- | 1,6 | A 01 N 43/08<br>C 07 D 307/32 |
| | US - A - 2 974 084 (R.L. MAYHEW)<br>* Insgesamt *<br>-- | 1,6 | |
| D | US - A - 3 488 732 (E.I. HEIBA)<br>* Zusammenfassung *<br>-- | 1,6 | |
| D | DE - A - 2 630 981 (I.C.I.)<br>* Seiten 1,2 *<br>---- | 1,6 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 25-09-1981 | MAISONNEUVE |